# EUROPEAN PATENT SPECIFICATION

(11) **EP 2 508 084 B1**
(45) Date of publication and mention of the grant of the patent: **08.01.2020**
(21) Application number: 10839868.6
(22) Date of filing: 25.11.2010
(51) Int. Cl.: A61K 35/64, A61K 36/734, A23L 21/20, A61K 35/63, A23L 33/105

(54) **BIOLOGICALLY ACTIVE FOOD ADDITIVE FOR TREATMENT OF MYOCARDIAL ISCHEMIA**
BIOLOGISCH WIRKENDER NAHRUNGSMITTELZUSATZ ZUR BEHANDLUNG VON MYOKARDIALER ISCHEMIA
ADDITIF BIOACTIF POUR ALIMENTS DESTINÉ AU TRAITEMENT DE L'ISCHÉMIE MYOCARDIALE

(30) Priority: 30.11.2009 RU 2009144334
(43) Date of publication of application: 10.10.2012
(73) Proprietor: Obshestvo S Ogranichennoj Otvetstvennostju "PARAFARM", Penza 440023 (RU)
(72) Inventor: ELISTRATOV, Dmitriy Gennadjevich, Penza 440060 (RU)
(74) Representative: Engel, Christoph Klaus
(86) International application number: PCT/RU2010/000704
(87) International publication number: WO 2011/078736

(56) References cited:
- RU-C1- 2 100 027
- RU-C1- 2 191 591
- RU-C1- 2 243 781
- FEDERALNY REESTR BIOLOGICHESKI AKTIVNYKH DOBAVOK K PISCHE 2005, page 401, 407, XP008164757
- KHISMATULLINA N. Z. APITERAPIYA., PERM: MOBILE 2005, pages 56 - 60, 136-137, XP008169119
- KRYLOV VASILY N ET AL: "Experimental study of Bee Royal Jelly cardioprotective characteristics", MELLIFERA, vol. 6, no. 10-12, 2006, pages 28-32, XP008177642, ISSN: 1302-5821
- MILLER A L: "Botanical influences on cardiovascular disease", ALTERNATIVE MEDICINE REVIEW, THORNE RESEARCH INC., SANDPOINT, US, vol. 3, no. 6, 1 December 1998 (1998-12-01), pages 421-431, XP002996497, ISSN: 1089-5159
- LONG S R ET AL: "Effect of hawthorn (Crataegus oxycantha) crude extract and chromatographic fractions on multiple activities in a cultured cardiomyocyte assay", PHYTOMEDICINE, GUSTAV FISCHER VERLAG, STUTTGART, DE, vol. 13, no. 9-10, 24 November 2006 (2006-11-24), pages 643-650, XP028022187, ISSN: 0944-7113, DOI: 10.1016/J.PHYMED.2006.01.005 [retrieved on 2006-11-24]

## Description

The invention relates to biologically active food additives and is intended for the treatment of myocardial ischemia. Known are therapeutic preparations in the form of phytopreparations for the treatment of patients suffering from various kinds of cardiovascular system disorders (RU 2000807, "CORDIS" Preparation for the Treatment of Cardiovascular Diseases"; RU 2019185, "Medicinal Plants Preparation with a Sedative and Hypotensive Action"; RU 2020952, "Glaya", "Darya", "Elena" Medicinal Plants Preparations with a General Health-Improving and Antisclerotic Action"; RU 2088250, "A Medicinal Preparation for Patients with Disordered Cardiac Rhythm"; RU 2104024, "Kasmin" Preparation for the Prevention and Treatment of Thromboses").

However, the above phytotherapeutic agents have a number of limitations. They belong to pharmaceuticals and therefore need to be prescribed and controlled by a physician, i.e., do not belong to preventive preparations having the status of a biologically active additive.

They include a rather large number of components which along with benefits arising from a versatile harmonic action on the body (Curative Preparations, Almaty, Kainar Publishers, 1991) also impose substantial constraints on the possibility to set up a wide-scale commercial production of such preparations. Therefore, multicomponent preparations often remain folk remedies.

By way of example, "Kasmin" according to RU 2104024, 10.02.1998 belongs to such preparation. It contains Aesculo hippocastano semenis, Glycyrrhizae radices, Menthae piperitae folia or Melissae officinalis herba, Rozae fructus and Crataegi fructus taken in a certain ratio and ground into 3-4 mm particles. The preparation acts not only on the hemostatic system itself but also on the cardiovascular system as a whole, nervous system, lipidic carbohydrate metabolism. It is administered to adults for the prevention and comprehensive treatment of thromboses with various etiology, including but not limited to thrombophlebitis, thromboembolic disorders in case of myocardial infarction, embolic apoplexies and the like, for the prevention of postoperative period thrombosis and also for the prevention of atherosclerosis.

The above preparation has a number of limitations, namely, contains a large number of ingredients which as noted above presents certain organizational and technological difficulties for mass production.

The preparation is intended for making an infusion dosage form so that the substances beneficial for a body are inevitably partly lost due to using only the liquid phase. In addition, it also involves a cumbersome process of preparing and inconvenience of administration for users.

In the Federal Register of biologically active food supplements "Federalny reestr biologicheski aktivnykh dobavok k pische" 2005, Pages 401, 407; several biologically active food supplements like "Apikardin" are registered.

In the paper by Krylov, Vasily N. et al. "Experimental Study of Bee Royal Jelly Cardioprotective Characteristics" in MELLIFERA, Vol. 6, No. 10-12, 2006, Pages 28-32, ISSN: 1302-5821, a cardioprotective effect of bee royal jelly usage has been investigated.

In the paper by Miller, A. L. "Botanical Influences on Cardiovascular Disease", in Alternative medicine review, THORNE RESEARCH Inc., Sandpoint, USA, Vol. 3, No. 6, 1 December 1998 (1998-12-01), Pages 421-431, ISSN: 1089-5159, the treatment of cardiovascular disease by botanicals like common hawthorn (Crataegus oxycantha) has been investigated.

It is an object of the invention to provide a preparation meeting the status of a biologically active food additive wherein the above limitations are overcome.

A biologically active food additive has to include a minimum amount of pharmacopeial ingredients with the most pronounced therapeutic properties with respect to the cardiovascular system with a minimum of contraindications and side effects.

A biologically active food additive has to ensure a maximum preservation of total biologically active substances upon ingestion, be easy to use and store and suitable for mass production.

The technical effect achieved using the invention consists in providing a dosed preparation meeting the status of a biologically active food additive, comprising a certain amount of high performance ingredients whose combined effect, on the one hand, ensures a long-lasting effective action. The technological aspects of the production of biologically active food additives present no difficulties for their mass production, and the consumer package enables sensory perception of the entire combination of biologically active substances and ease in use and storage.

A biologically active food additive for the treatment of myocardial ischemia is provided, comprising hawthorn flowers and/or berries and/or leaves, royal jelly and an excipient in a following ratio of ingredients:
hawthorn flowers and/or berries and/or leaves: from 61 to 90 wt. %,
royal jelly: from 10 to 24 wt.%,
excipients: from 0 to 29 wt.%.

The inventive preparation is in the powdered, tableted or capsulated form and may also be in the form of an aqueous alcoholic extract and dosage forms prepared on the basis of such extract, namely, powder, tablet and capsule.

The selection of hawthorn and royal jelly as ingredients of the biologically active food additive is associated with the following reasons.

Hawthorn is one of the primary plants used in the phytotherapeutic practice for regulating the cardiovascular activity both independently (see for example RU 2085206, "An Agent for the Treatment of Paroxysmal Tachycardia and Angioneurosis) and as part of phytopreparations (see the above referenced patent documents).

Hawthorn fruits contain tanning agents, pectins, flavonoids (quercetin, hyperoside, vitexin), anthocyans and leucoanthocyanin triterpene and phytosterol-like compounds, choline, acetylcholine, ursolic, chlorogenic, caffeic, crataegus, oleanolic acids, fatty oil, sugars, trace elements such as copper, cobalt, zinc, manganese, iron, molybdenum, and others, vitamins C, PP, B1, B2, carotenoids. Such formulation ensures a high efficiency of fruits against various diseases, especially cardiac diseases (Minaeva V. G., Medicinal Plants of Siberia, Novosbirsk, Nayka Publishers, 1991, 431 p.).

Hawthorn flowers and leaves contain flavonols (quercetin, quercitrin, hyperoside), essential oil, caffeic and chlorogenic acids, acetylcholine, choline and the same trace elements as in the fruits.

Hawthorn preparations have a versatile systemic action, act practically on the entire cardiovascular system, have an antiarrhythmic (cardiac arrhythmia is a disorder of the cardiac rate, rhythm and normal coordinated reflex between various cardiac parts and areas), cardiostimulatory, coronary dilatory, hypotensive, antiatherosclerotic, antistenocardic, antitachycardic and sedative action. Hawthorn flavonoids suppress the action of the angiotensin converting enzyme (ACE) responsible for vasoconstriction. The vasodilatory action creates conditions for a smooth reduction in the arterial pressure and myocardium load, has a mild diuretic action. Data is available in support of the hawthorn action against the atherosclerotic process in the walls of arteries. Hawthorn improves oxygen supply of the cerebral neurons. Therefore, the hawthorn preparations are indicated in case of the age-related changes of the cardiovascular system function and may be to the full extent referred to the agents improving the quality of life in the elderly and old age. Hawthorn preparations are prescribed against angioneurosis, ciliary arrhythmia, paroxysmal tachycardia, hypertension, stenocardia, extrasystole, cardiovascular insufficiency, thyrotoxicosis, atherosclerosis, insomnia, during climacterical period, in case of hypoimmunity (M.Risman, Biologically Active Additives: the Unknown about the Known, Translated from English, Moscow, Art Business Center Publishers, 1998).

As the second ingredient, royal jelly is used in the invention. Royal jelly is an agent widely used in the folk and scientific medicine (included in the State Pharmacopeia). It has long been considered as a kind of a vital elixir and used against various diseases. Its chemical composition is so unique that one may hardly find another equally curative product. Royal jelly contains about 400 substances essential to a human body with some of them (for example, oxidecenoic acids) being naturally found only in royal jelly. The main ones of them are essential fatty and amino acids, proteins, trace elements, natural antibiotics, hormone-like substances, vitamins and enzymes. Their proportions are selected so as to ensure forced metabolism directed to the development of the body structures and functions (Krivtsov N,I., Burmistrova L.A., Apitherapy Theory and Agents, Moscow).

Although the components of the inventive preparation are known in the folk and traditional medicine, their combination in a single product in the claimed ratios is not disclosed in the prior art.

Thus, it has been unexpectedly found that the biologically active additive comprising hawthorn flowers and/or berries and/or leaves in amounts of from 61 to 90 wt. % and royal jelly in amounts of 10 to 24 wt.% exhibits a prolonged, i.e., long-lasting positive effect on the human cardiovascular system.

This unexpected effect of the use of royal jelly and hawthorn in the claimed ratios is explicable by the following. In the therapy of heart diseases, it is important to consider that they are often associated with or determined by other causes, for example, hypertension, renal insufficiency, etc. Therefore, royal jelly action may be associated with a beneficial effect above all on the general course of a pathological process being the main cause of the heart disease and not on myocardium.

There is sufficient amount of data from the literature regarding the influence of royal jelly on these general systemic pathological processes (Lupachev V., Apilac in the Treatment of Coronary Atherosclerosis, Ryazan, 1965; Ludyansky E.A. et al., Apilac in Neurological Patients with Vascular Crises and Asthenia. Apitherapy Today, 2nd Collected Volume, Rybnoye, 1993; Lyusov V.A. et al., Influence of Royal Jelly on the Course of Stenocardia and lipid blood composition in the patient with ischemic heart disease//Apitherapy Today (3rd Collected Volume) Rybnoye,1994).

In other words, hawthorn preparations act on the "consequence" and royal jelly on the "causes" of cardiovascular diseases. Precisely this multidirectional action accounts for a high efficiency of the claimed preparation for a long time. At the same time, the maximum depot action of the claimed additive is achieved namely due to the claimed ratio of hawthorn and royal jelly having been found by applicants.

It should be noted that the cardioprotective action of the claimed agent is higher than that of its separate parts due to the antioxidant activity of royal jelly leading to an antihypoxic effect in case of myocardial ischemia.

The use of this biologically active food additive will make it possible to achieve a more lasting effect than using chemical pharmaceuticals. It is necessary to remember that this preparation is not an instantaneous action agent and is unable to relieve, for example retrosternal pain or normalize the heart rhythm. It is a long-lasting action agent for the prevention and systematic comprehensive treatment. It is very important that no contraindications or side effects have been identified even in case of a very long application (over one year).

Examples of preparing a biologically active food additive:

### Example 1

Ground hawthorn fruits and royal jelly were provided in the following ratio: hawthorn fruits: 90 wt. %, royal jelly: 10 wt. %. The components were mixed together to homogeneity and the final product was encapsulated.

### Example 2

Ground hawthorn fruits and leaves, royal jelly and excipient (calcium stearate, sugar, starch) were provided in the following ratio: hawthorn fruits and leaves: 61 wt. %, royal jelly: 24 wt. %, excipients: 15 wt. %. The components were mixed together to homogeneity and the final product was tableted.

## Claims

1. A biologically active food additive for use in the treatment of myocardial ischemia, **characterized in that** it comprises hawthorn flowers and/or berries and/or leaves, royal jelly and excipient in a following ratio of ingredients:
- hawthorn flowers and/or berries and/or leaves: from 61 to 90 wt. %,
- royal jelly: from 10 to 24 wt.%,
- excipients: from 0 to 29 wt.%.

## Patentansprüche

1. Biologisch aktiver Nahrungsmittelzusatz zur Verwendung bei der Behandlung von Myokardischämie, **dadurch gekennzeichnet, dass** er Weißdornblüten und/oder -beeren und/oder -blätter, Gelee Royale und Trägerstoffe in folgendem Verhältnis der Inhaltsstoffe enthält:
- Weißdornblüten und/oder -beeren und/oder -blätter: von 61 bis 90 Masse %
- Gelee Royale: von 10 bis 24 Masse %
- Trägerstoffe: von 0 bis 29 Masse %

## Revendications

1. Additif alimentaire biologiquement actif destiné à être utilisé dans le traitement de l'ischémie myocardique, **caractérisé en ce qu'**il contient des fleurs et/ou des baies et/ou des feuilles d'aubépine, de la gelée royale et des excipients, dans le ratio d'ingrédients suivant :
- fleurs et/ou baies et/ou feuilles d'aubépine : de 61 à 90 % pds,
- gelée royale : de 10 à 24 % pds,
- excipients : de 0 à 29 % pds.
